# EUROPEAN PATENT APPLICATION

(11) **EP 2 042 138 A1**
(43) Date of publication of application: **01.04.2009**
(21) Application number: 07790802.8
(22) Date of filing: 13.07.2007
(51) Int. Cl.: A61F 13/02, A61K 9/70

(54) **PATCH AND PATCH PREPARATION**

(30) Priority: 13.07.2006 JP 2006192346
(71) Applicant: NITTO DENKO CORPORATION, Ibaraki-shi, Osaka 567-8680 (JP)
(72) Inventor: HARIMA, Jun c/o Nitto Denko Corporation, Osaka 567-8680 (JP); SAKURABA, Ryouhei c/o Nitto Denko Corporation, Osaka 567-8680 (JP)
(74) Representative: Grünecker, Kinkeldey, Stockmair & Schwanhäusser Anwaltssozietät
(86) International application number: PCT/JP2007/064022
(87) International publication number: WO 2008/007786

(57) **Abstract**

The present invention relates to a patch including a backing, a pressure-sensitive adhesive layer formed on one surface of the backing, and a liner having a thickness of T and laminated on the pressure-sensitive adhesive layer, in which the liner has a groove formed from the surface opposite to the surface on which the pressure-sensitive adhesive layer is laminated and having a depth of from T/2 to less than T, the groove has a planar shape which enables the liner to be divided into two or more liner pieces by the groove, and the liner has a bending resistance before forming the groove of 50 mm or more.

## Description

### TECHNICAL FIELD

The present invention relates to a patch including a backing, a pressure-sensitive adhesive layer formed on one surface of the backing and a liner laminated on the pressure-sensitive adhesive layer, and a patch preparation containing a drug in the pressure-sensitive adhesive layer.

### BACKGROUND ART

In recent years, various patches and patch preparationes has been developed. These patches and patch preparationes are very excellent in view of protection of a wound and continuous transdermal administration of a drug. In the patch or patch preparation, a pressure-sensitive adhesive layer is laminated on a backing formed of a fabric or a plastic film, and a liner is laminated on the pressure-sensitive adhesive layer. Such a liner not only protects the exposed surface of the pressure-sensitive adhesive layer but also, when used for a patch or patch preparation using a flexible backing, exerts an effect of improving the self-holding properties as a patch or patch preparation and enhancing the handleability of the patch or patch preparation. In using such a patch or patch preparation, the user peels off the liner from the pressure-sensitive adhesive layer and applies the appeared pressure-sensitive adhesive layer with pressure to the application site of a patient. Accordingly, it is preferred that the liner can be easily separated from the pressure-sensitive adhesive layer.

JP-A-2003-033389 (Patent Document 1) discloses a patch material including a pressure-sensitive adhesive layer having a thickness of R and formed on one surface of a backing and a liner having a thickness of T and laminated on the pressure-sensitive adhesive layer, in which a groove is formed in the liner, the groove width is 200 µm or less, and the groove depth is from 14T/15 to less than (T+R).

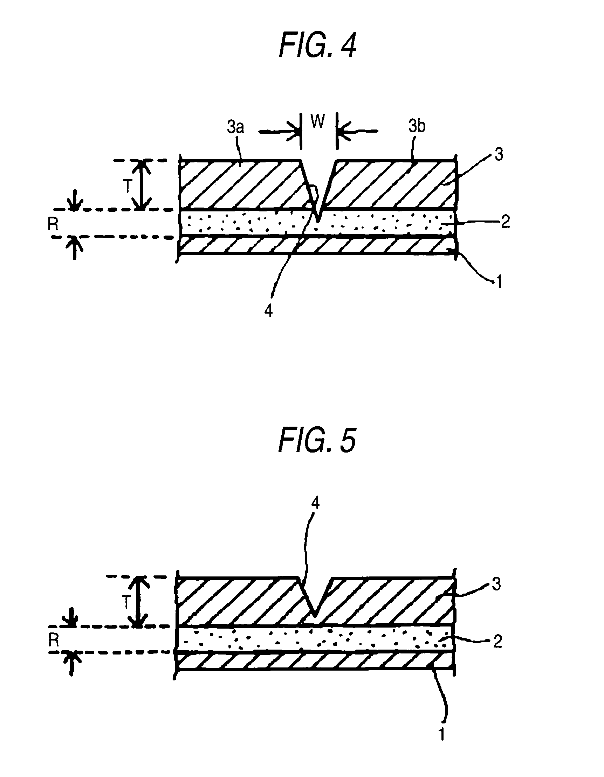

Fig. 4 is a cross-sectional view of one example of the patch material above. This patch material includes a backing 1, a pressure-sensitive adhesive layer 2 formed on one surface of the backing, and a liner 3 laminated on the pressure-sensitive adhesive. In this patch material, a groove 4 is formed in the liner 3. The groove 4 divides the liner 3 into a liner piece 3a and a liner peace 3b. In use, the user usually folds the patch material along the groove 4 and then separates the liner 3 by lifting the end part of the liner piece 3a or liner piece 3b at the groove 4. In this way, the groove 4 improves the peelability of the liner 3 and thereby enhances the handleability when using the patch material. Such a groove can be formed on the liner by laser processing, blade processing or the like.

However, in the example of Fig. 4, the groove 4 reaches the pressure-sensitive adhesive layer 2 to expose the pressure-sensitive adhesive layer 2 and therefore, the grooving needs to be more devised so as to prevent contamination of the pressure-sensitive adhesive layer 2. Also, the laser processing, blade processing or the like causes a mechanical damage in the pressure-sensitive adhesive layer, such as partial chipping of the pressure-sensitive adhesive layer 2. Particularly, in the case of laser processing, a thermal damage is sometimes produced in the pressure-sensitive adhesive layer.

Furthermore, depending on the component or amount of the pressure-sensitive adhesive layer, a pressure-sensitive adhesive layer component such as additive may bleed out from the groove 4 during storage of the patch material. In the case where the pressure-sensitive adhesive layer contains a drug or the like, sublimation or decomposition of the drug or the like may occur. That is, there is room to improve the temporal stability of the pressure-sensitive adhesive layer. In addition, resulting from bleeding out of a pressure-sensitive adhesive component such as additive from the groove 4 during storage of the patch material, which may occur depending on the component of the pressure-sensitive adhesive layer, the patch material sometimes adheres to the packaging material enclosing therein the patch material and can be hardly taken out from the packaging material. Accordingly, there is room to devise means for solving such a problem.

Furthermore, in the example of Fig. 4, since the groove 4 reaches the pressure-sensitive adhesive layer 2, the liner 3 is divided into a liner piece 3a and a liner piece 3b and is not connected at the groove bottom. Accordingly, there may be envisaged a case where the self-holding property as a patch material is not sufficient, and this may leave room for improvement in the handleability as a patch material.

On the other hand, JP-A-2003-033389 also discloses a patch material where the groove 4 does not reach the pressure-sensitive adhesive layer 2, and Fig. 5 shows a cross-sectional view of the patch material. In this example, the liner is connected at the bottom of the groove 4, and the depth of the groove 4 is from 14T/15 to less than T, where T is the liner thickness. In this publication document, it is also indicated that the material used for the liner 3 is not particularly limited and various materials may be used.

Taking notice of the method of using such a patch material, the user is usually required to perform the following three operations for separating the liner 3:
(i) cutting the connected part of the liner 3 at the groove 4 bottom to divide the liner into a liner piece 3a and a liner piece 3b;
(ii) folding the patch material along the groove 4; and
(iii) lifting the end part of the liner piece 3a or liner piece 3b at the groove 4 to thereby separate the entire liner.

As described above, with respect to the example shown in Fig. 5, in the above mentioned publication document, the properties of the liner 3 are not specified as means for peeling off the liner at the groove bottom and the mere depth of the groove 4 is only specified. Therefore, regarding this example of this publication document, it may be envisaged that depending on the property of the liner 3, the liner at the groove bottom cannot be easily cut, making it difficult to peel off the liner. In fact, as to the operation for cutting the connected part of the liner 3 in (i) above, this publication document uses an expression that the liner 3 is "ruptured" or "torn" into liner pieces. Accordingly, it is understood that in the example of Fig. 5, the operations (i) and (ii) are not achieved by a series of operations.

Also, in the example of Fig. 5 of this publication document, the depth of the groove 4 is from 14T/15 to less than T. However, when the depth of the groove 4 is controlled to such a very narrow range as in this example, a new difficulty may arise in the industrial mass production.

Furthermore, in this publication document, it is stated that the thickness of the liner 3 in the connected part at the groove bottom is preferably as thin as possible. This implies a difficulty in employing a wider range for the depth of the groove 4.

Surprisingly, it has been found that when a certain kind of a liner according to the present invention is employed, complicated operations such as (i) and (ii) above are not necessarily required and only by causing the user to merely fold the patch material along the groove into a mountain shape with the groove as the ridge, the liner at the groove bottom can be easily broken to separate the liner.

Furthermore, surprisingly, in the case of employing such a liner according to the present invention, it is not necessarily required to control the depth of the groove 4 to such a very narrow range as from 14T/15 to less than T.

Also, with respect to the example of Fig. 5, in this publication document, the properties of the liner 3 in the patch material are not particularly specified. Accordingly, there may be envisaged a case where depending on the liner employed, such a patch material is still insufficient in the self-holding property as a patch material, and this leaves room for improvement in the handleability as a patch material.
Patent Document 1 : JP-A-2003-033389

### DISCLOSURE OF THE INVENTION

Under these circumstances, an object of the present invention is to provide a patch and a patch preparation, where the pressure-sensitive adhesive layer has high temporal stability without causing contamination, mechanical damage or thermal damage in the pressure-sensitive adhesive layer until immediately before use and where the shape as a patch and a patch preparation can be easily maintained and the liner can be easily separated so as to ensure excellent handleability.

Unexpectedly, when a certain groove is formed in the liner of the patch or patch preparation and the bending resistance of the liner is specified to a certain value, a patch and a patch preparation each enabling easy separation of the liner only by folding the patch along the groove can be obtained.

That is, the present invention provides the followings.
(1) A patch comprising a backing, a pressure-sensitive adhesive layer formed on one surface of the backing, and a liner having a thickness of T and laminated on the pressure-sensitive adhesive layer, wherein the liner has a groove formed from the surface opposite to the surface on which the pressure-sensitive adhesive layer is laminated and having a depth of from T/2 to less than T, the groove has a planar shape which enables the liner to be divided into two or more liner pieces by the groove, and the liner has a bending resistance before forming the groove of 50 mm or more.
(2) The patch according to (1) above, wherein the groove has a width at the bottom of 200 µm or less.
(3) The patch according to (2) above, wherein the groove has a cross-sectional shape of a substantially U-shape or a substantially V-shape.
(4) A patch preparation comprising the patch according to any one of (1) to (3) above, wherein the pressure-sensitive adhesive layer contains a drug.

In the patch and patch preparation of the present invention, the liner has a groove formed from the surface opposite to the surface on which the pressure-sensitive adhesive layer is laminated and having a depth of less than T. Here, T is the liner thickness. That is, the groove does not reach the pressure-sensitive adhesive layer, and the liner is connected at the groove bottom. This allows the liner to protect the pressure-sensitive adhesive layer even at the groove bottom and therefore, the pressure-sensitive adhesive layer is protected by the liner and is not exposed to the environment at the groove bottom. As a result, bleeding out of a pressure-sensitive adhesive layer component such as additive from the groove part does not occur during storage of the patch. Particularly, in the case of a patch preparation containing a drug or the like in the pressure-sensitive adhesive layer, sublimation or decomposition of the drug or the like is reduced. In other words, the temporal stability of the pressure-sensitive adhesive layer is high. Also, the groove formation involves neither contamination of the pressure-sensitive adhesive layer nor a mechanical damage of the pressure-sensitive adhesive layer by laser processing, blade processing or the like, such as chipping of the pressure-sensitive adhesive layer. Furthermore, in forming the groove by laser processing, a thermal damage is not produced in the pressure-sensitive adhesive layer.

Furthermore, in the patch and patch preparation of the present invention, since the pressure-sensitive adhesive layer is protected by a liner, when the patch or patch preparation is enclosed in a packaging material and stored, a pressure-sensitive adhesive layer component such as additive can be prevented from bleeding out from the groove portion and causing the patch or patch preparation to attach to the packaging material enclosing it and in turn, the patch or patch preparation can be easily taken out from the packaging material.

In the patch and patch preparation of the present invention, the groove depth is T/2 or more and the bending resistance of the liner before forming the groove is 50 mm or more. By virtue of such a cross-sectional shape of the groove, combined with setting the bending resistance of the liner to 50 mm or more, the liner at the groove bottom can be broken only by merely folding the patch or patch preparation along the groove in using the patch or patch preparation. Furthermore, the groove has a planar shape which enables the liner to be divided into two or more liner pieces by the groove and therefore, dividing one piece of liner into one liner piece or two or more liner pieces by the groove (hereinafter, this operation is referred to as "liner division") can be quite easily achieved. Subsequently, the user lifts the end part of the divided liner piece, whereby the liner can be very easily separated. In this way, the handleability as a patch and a patch preparation is excellent in terms that the separation of the liner can be very easily attained by a series of operations.

Also, in the patch and patch preparation of the present invention, the groove depth is less than T and the bending resistance of the liner is 50 mm or more, which makes it very easy to maintain the shape as a patch and a patch preparation. In this respect, the handleability as a patch and a patch preparation is excellent.

Furthermore, in the patch and patch preparation of the present invention, the groove depth is from T/2 to less than T, so that the range of the groove depth can be made relatively wide and a groove can be easily formed in the industrial mass production.

### BRIEF DESCRIPTION OF THE DRAWINGS

Fig. 1 is a cross-sectional view of the patch in one embodiment of the present invention.
Fig. 2 is a cross-sectional view of the patch in another embodiment of the present invention.
Fig. 3 is a graph showing the stability with respect to the drug content.
Fig. 4 is a cross-sectional view of the patch as a conventional example.
Fig. 5 is a cross-sectional view of the patch as another conventional example.

### Description of Reference Numerals and Signs

- 1: backing
- 2: pressure-sensitive adhesive layer
- 3: liner
- 3a: liner piece
- 3b: liner piece
- 4: groove
- R: thickness of pressure-sensitive adhesive
- T: thickness of liner
- W: width at groove top
- Y: thickness of connected part
- Z: width at groove bottom

### BEST MODE FOR CARRYING OUT THE INVENTION

The present invention is described below by referring to the drawings attached.

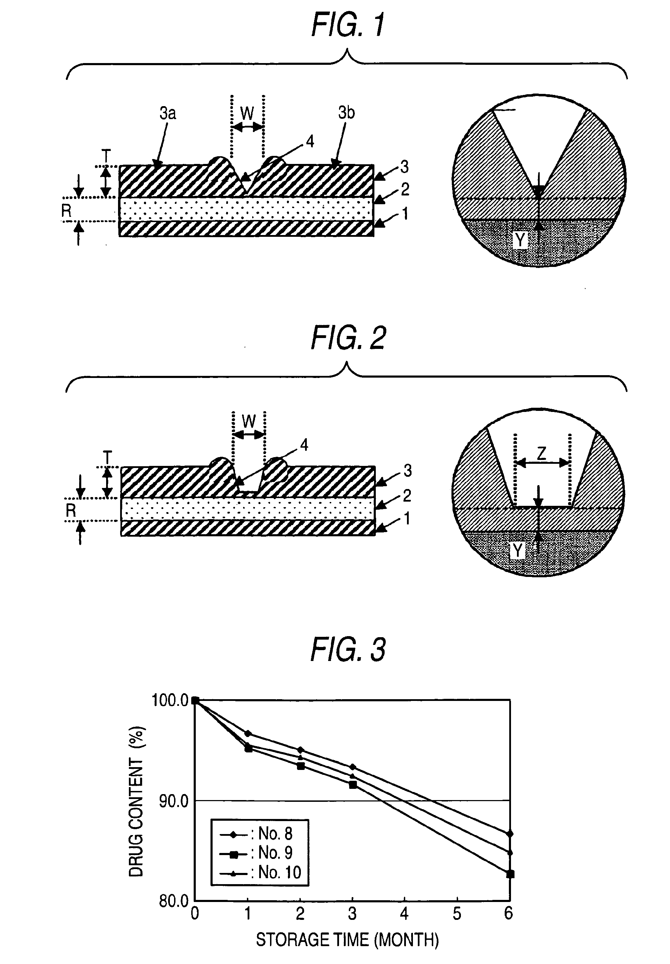

Fig. 1 is a cross-sectional view of the patch in one embodiment of the present invention. This patch includes a backing 1, a pressure-sensitive adhesive layer 2 having a thickness of R and formed on one surface of the backing, and a liner 3 having a thickness of T and laminated on the pressure-sensitive adhesive layer.

The bending resistance of the liner 3 before forming a groove needs to be 50 mm or more. If the bending resistance is less than 50 mm, the liner is too soft and the liner division may not be effected. As the bending resistance of the liner is larger, the user can effect the liner division by a smaller motion. From this standpoint, the bending resistance of the liner is preferably 70 mm or more, and a larger bending resistance of the liner is more preferred. However, in the case of using a normal liner material, the bending resistance of the liner and the thickness of the liner are substantially in a proportional relation, and an excessively large thickness of the liner is disadvantageous in view of production of the patch. Also, since the liner is stripped and discarded in use, if the thickness of the liner is excessively large, this is disadvantageous in view of the cost and furthermore, the portability of the patch or the handleability in use sometimes becomes poor. For this reason, the bending resistance of the liner is preferably 200 mm or less.

The value of bending resistance as referred to in the present specification means a value measured on the liner before groove formation, based on the description in Japanese Industrial Standards, "JIS L1085 5.7 Method A of Bending Resistance (45° cantilever method)".

The liner 3 is not particularly limited so long as it has the above-described bending resistance, but the examples of the liner includes a plastic film such as polyester film, particularly polyethylene terephthalate film, and a laminate film thereof. A polyester film, particularly a polyethylene terephthalate film, is preferred because of a large number of types, an appropriate thickness as a patch, and ease in selecting a material having the bending resistance above. Considering easy processing applicability and processing accuracy, a film having a uniform thickness is preferred. The thickness is not particularly limited but is preferably from 25 to 200 µm, and more preferably from 50 to 150 µm, in view of easy production of the patch, the cost of the liner, and the portability, handleability or the like of the patch.
the surface of the liner which is to face the pressure-sensitive adhesive layer is usually subjected to a releasant treatment in order to enable the liner to be more easily peeled off from the pressure-sensitive adhesive layer.

The depth of the groove 4 formed in the liner 3 from the surface opposite to the surface on which the pressure-sensitive adhesive layer 2 is laminated needs to be T/2 or more. If the groove depth is less than T/2, the liner division may fail when using the patch and the handleability at the separation of the liner is bad. From the standpoint of enabling efficient liner division and thereby improving the handleability, the groove depth is preferably 2T/3 or more.

An enlarged view of the groove 4 bottom is shown in the circle of Fig. 1. In the present invention, it is necessary to positively leave the thickness of the connected part, indicated by Y in the circle of Fig. 1, in the liner. In other words, the groove depth needs to be less than T. If the groove depth is T or more, the groove reaches the pressure-sensitive adhesive layer and at the groove formation, the pressure-sensitive adhesive layer comes into contact with a blade (e.g., die roll or razor), a laser or the like, as a result, there may be produced contamination in the pressure-sensitive adhesive layer or a mechanical damage in the pressure-sensitive adhesive layer. Also, since the pressure-sensitive adhesive layer is exposed to the environment, the temporal stability of the pressure-sensitive adhesive layer may not be ensured. Furthermore, since the pressure-sensitive adhesive layer is in a state of being partially exposed, the pressure-sensitive adhesive layer or an additive contained therein may bleed out from the groove. From the standpoint of efficiently avoiding such a problem, the groove depth is preferably less than 14T/15.

Examples of the cross-sectional shape of the groove 4 include a substantially V-shape, a substantially rectangular shape, a substantially U-shape, and a shape where the side or angle of such a shape is partially curved or distorted. In view of enabling efficient liner division and improving the handleability, the cross-sectional shape is preferably a substantially V-shape or a substantially U-shape, and more preferably a substantially V-shape.

In the patch shown in Fig. 1, the groove 4 has a substantially V-shaped cross-section, and the tip thereof has an acute cross-sectional shape. Such a cross-sectional shape is preferred because by virtue of the acute tip of the groove 4, the liner division can be performed with a light force in using the patch. The tip angle of the groove 4 is preferably 45° or less.

The width W at the top of the groove 4 is not particularly limited, but the width at the top of the groove 4 is preferably 300 µm or less, and more preferably 250 µm or less. By setting the width W at the top of the groove 4 to 300 µm or less, the touch feeling becomes better. In view of touch feeling, a smaller width W at the top of the groove is more preferred, but from the standpoint of liner visibility, the width is preferably 1 µm or more.

The term "width at the top of the groove" as used herein means the width of the groove as measured on the liner surface, and is indicated by W in the example of Fig. 1.

The width at the bottom of the groove 4 is not particularly limited but is preferably 200 µm or less, and more preferably 100 µm or less, from the standpoint of efficiently preventing the liner division before use.

The width at the bottom of the groove as used herein means the width in the deepest part of the groove, and in the case of a groove which does not have a deepest-part region having a nearly flat surface as in the embodiment of Fig. 1, the width of the bottom of this groove is substantially 0 µm.

The values indicating the cross-sectional shape such as groove width and groove depth as used in the present specification mean values measured using a microscope (manufactured by Keyence Corp.). The thickness of the connected part means a value calculated by subtracting the groove depth from the liner thickness.

The planar shape of the groove is a shape which enables the liner to be divided into two or more liner pieces by the groove. By virtue of such a planar shape, when the liner at the groove bottom is broken as described above, it becomes quite easy to produce a plurality of liner pieces, that is, to divide the liner. There are various kinds of such planar shapes of groove. Examples thereof include those which extend from a first position in an edge of the liner to a second position in another edge of the liner. It is preferred that the groove have a planar shape in which the first position differs from the second position, from the standpoints of enabling efficient liner division and easy liner removal and enabling the patch to have satisfactory handleability. More specifically, in the case where the liner has a nearly rectangular shape, examples of the planar shape of the groove include a nearly straight line and a curve such as wavy line, which extend from a certain position in one side of the liner, in particular from an approximate center of the side, to a certain position in the side which is opposed to that side, in particular to an approximate center of this side. From the standpoint of easy production, a nearly straight line is preferred. Use of a wavy line has an advantage that immediately after liner division, wave crest parts of the liner lift up from the pressure-sensitive adhesive layer and these parts can be used as a pinching area to easily peel off the liner.

The backing 1 is not particularly limited and examples thereof include various plastic films, nonwoven fabrics, paper, woven fabrics, knitted fabrics, metal foils, and laminates of these. According to need, a metal such as aluminum may be vapor-deposited on these materials.

The plastic films are not particularly limited. Examples thereof include various films made of poly(vinyl chloride) alone, copolymers of a monomer such as ethylene, propylene, vinyl acetate, acrylic acid, an acrylic ester, methacrylic acid, a methacrylic ester, acrylonitrile, styrene, or vinylidene chloride and one or more other monomers, olefin polymers such as polyethylene, polypropylene, and ethylene/vinyl acetate copolymers, polyester polymers such as poly(ethylene terephthalate) and polyether polyesters, and polyamide polymers such as polyether/polyamide block polymers.

The thickness of the backing is usually from 10 to 500 µm, and preferably from 10 to 200 µm.

The pressure-sensitive adhesive to be used for forming the pressure-sensitive adhesive layer 2 is not particularly limited. Examples thereof include acrylic pressure-sensitive adhesives containing an acrylic polymer; rubber pressure-sensitive adhesives such as styrene/diene/styrene block copolymers (e.g., styrene/isoprene/styrene block copolymers and styrene/butadiene/styrene block copolymers), polyisoprene, polyisobutylene, and polybutadiene; silicone pressure-sensitive adhesives such as silicone rubbers, dimethylsiloxane-based polymers, and diphenylsiloxane-based polymers; vinyl ether pressure-sensitive adhesives such as poly(vinyl methyl ether), poly(vinyl ethyl ether), and poly(vinyl isobutyl ether); vinyl ester pressure-sensitive adhesives such as vinyl acetate/ethylene copolymers; and polyester pressure-sensitive adhesives produced from a carboxylic acid ingredient such as dimethyl terephthalate, dimethyl isophthalate, or dimethyl phthalate and a polyhydric alcohol ingredient such as ethylene glycol.

The thickness of the pressure-sensitive adhesive layer is usually from 10 to 200 µm, and preferably from 15 to 150 µm.

The pressure-sensitive adhesive may be optionally subjected to a physical crosslinking treatment by the irradiation with radiation, such as irradiation with ultraviolet ray or electron beam, or a chemical crosslinking treatment using various crosslinking agents such as isocyanate-based compound (e.g., trifunctional isocyanate), organic peroxide, organic metal salt, organic alcoholate, metal chelate compound and polyfunctional compound (for example, a polyfunctional external crosslinking agent or a polyfunctional internal crosslinking monomer, e.g., diacrylate, dimethacrylate).

As described above, in the present invention, the thickness of the connected part, indicated by Y in Fig. 1, is positively left in the groove part of the liner. Accordingly, from the standpoint that an additive is often added to the pressure-sensitive adhesive layer and the additive can be unfailingly prevented from bleeding out, the patch of the present invention is particularly suitable for a case where an acrylic pressure-sensitive adhesive or a rubber pressure-sensitive adhesive is used as the pressure-sensitive adhesive.

The acrylic pressure-sensitive adhesive includes an acrylic acid ester pressure-sensitive adhesive in which the main component is a polymer containing a (meth)acrylic C₂₋₁₈ alkyl ester as a polymerization component. In view of good adhesion to the human skin and easy repetition of adhesion and separation, a copolymer obtained by copolymerizing 2-ethylhexyl acrylate as the (meth)acrylic acid alkyl ester, acrylic acid and N-vinyl-2-pyrrolidone in a weight ratio of (from 40 to 99.9)/(from 0.1 to 10)/(from 0 to 50) is preferred.

The rubber pressure-sensitive adhesive includes a rubber pressure-sensitive adhesive containing, as the main component, at least one member selected from a polyisobutylene, a polyisoprene and a styrene-diene-styrene copolymer. A pressure-sensitive adhesive obtained by blending a high molecular-weight polyisobutylene having a viscosity average molecular weight of 500,000 to 2,100,000 and a low molecular-weight polyisobutylene having a viscosity average molecular weight of 10,000 to 200,000 in a weight ratio of 95/5 to 5/95 is preferred, because the drug stability is high and both the required adhesive force and cohesive force can be satisfied.

If desired, the patch of the present invention can be prepared as a patch preparation by incorporating a drug into the pressure-sensitive adhesive layer. The drug is preferably a percutaneously absorbable drug. As described above, in the present invention, since the thickness of the connected part, indicated by Y in Fig. 1, is positively left in the groove part of the liner, the pressure-sensitive adhesive layer is not exposed to the environment at the groove part. Accordingly, the patch preparation of the present invention containing a drug in the pressure-sensitive adhesive layer is advantageous particularly in terms that the temporal stability of the drug is excellent.

For example, in order to control the adhesiveness and accelerate the percutaneous absorption of the drug, an additive may be optionally incorporated into the pressure-sensitive adhesive layer. The additive is not particularly limited and examples thereof includes an aliphatic acid ester composed of a higher fatty acid having a carbon number of 12 to 16 and a lower monohydric alcohol having a carbon number of 1 to 4. Examples of the higher fatty acid having a carbon number of 12 to 16 include a lauric acid, a myristic acid and a palmitic acid, and examples of the lower monohydric alcohol having a carbon number of 1 to 4 include a methyl alcohol, an ethyl alcohol, a propyl alcohol and an isopropyl alcohol.

Even when the additive is added in a large amount of 25 wt% to less than 100 wt%, particularly from 40 wt% to less than 70 wt%, to the pressure-sensitive adhesive layer 2 so as to control the pressure-sensitive adhesive force, by virtue of the above-described cross-sectional shape of the groove 4, the handleability and stability can be ensured while preventing bleeding out of the pressure-sensitive adhesive or additive from the groove 4.

Here, referring to Fig. 2, the patch shown herein has the same construction as the patch of Fig. 1 except that a substantially rectangular-shaped groove 4 with the groove width at the top being wider than the groove width at the bottom is formed in the liner. An enlarged view of the groove 4 bottom is shown in the circle of Fig. 2. In this embodiment, the bottom of the groove is substantially planar. In this case, the width Z at the groove bottom is 200 µm or less, and preferably 100 µm or less. In the case where the groove bottom is substantially planar, from the standpoint of efficiently preventing liner division before use, Z is preferably 200 µm, and more preferably 100 µm. This patch may also be prepared as a patch preparation by incorporating the above-described drug into the pressure-sensitive adhesive layer.

The patch and mediated patch described above are produced, for example, in the following manner. A liner is prepared, and a pressure-sensitive adhesive layer is laminated on one surface of the liner. A backing is then laminated on the pressure-sensitive adhesive layer. Alternatively, a backing is prepared, and a pressure-sensitive adhesive layer is laminated on one surface of the backing. A liner is then laminated on the pressure-sensitive adhesive layer. Techniques for the lamination are not particularly limited. Examples thereof include coating, adhesion, melt bonding, and fusion bonding.

In the case of a patch preparation, that is, in the case of incorporating a drug into the pressure-sensitive adhesive layer, examples of methods for drug incorporation include the mixing of a pressure-sensitive adhesive with a drug and the application and infiltration of a drug to the surface of a pressure-sensitive adhesive layer.

A groove having a depth of T/2 to less than T is formed on the liner surface before, during and/or after laminating the liner. Herein, T is the liner thickness. The groove formation method include, for example, blade processing by a die roll or a razor, and laser processing. Examples of the laser processing include a method using a CO₂ laser or a YAG laser. The planar shape of the groove having the cross-sectional shape above includes a substantially straight line and a curved line such as serpentine curve (e.g., wavy line).

The conditions of the laser processing vary depending on the material or thickness of the liner applied, and a groove having a desired cross-sectional shape can be easily formed by adjusting the laser output or the drug feed rate (or laser beam scan rate).

Finally, the method of using the patch and patch preparation of the present invention is described by referring to the embodiment of Fig. 1. In using the patch and patch preparation of the present invention, the user performs the following operations:
(1) folding the patch or patch preparation along the groove 4 into a mountain shape with the groove 4 as the ridge; by this operation, the groove connected part shown in the circle of Fig. 1 is "snap" broken and divided into a liner piece 3a and a liner piece 3b; and
(2) separating the liner by lifting the end part of the liner piece 3a or liner piece 3b at the groove 4.

On the other hand, the conventional patch material, for example, shown in Fig. 5 requires the following three operations:
(i) "tearing" the connected part of the liner 3 in the groove 4 bottom to separate the liner into a liner piece 3a and a liner piece 3b;
(ii) folding the patch material along the groove 4 into a mountain shape with the groove 4 as the ridge; and
(iii) separating the entire liner by lifting the end part of the liner piece 3a or liner piece 3b at the groove 4.

As understood, the patch and patch preparation of the present invention are assured of excellent handleability in that the operations (i) and (ii) above in the conventional technique can be completed only by operation (1) above and the liner can be very easily separated by a series of operations (1) and (2) above.

### EXAMPLES

The present invention is described in greater detail below based on Examples, but the present invention is not limited thereto. Unless otherwise indicated, the "parts" means "parts by weight".

### (Example 1)

### Groove Formation in Liner by Itself:

Various polyethylene terephthalate films (PET) having a bending resistance and a thickness shown in Table 1 were used as the liner. A groove was formed in the liner by using a laser cutting device (manufactured by Daisho Kagaku Kikai Kogyo K.K.) having incorporated thereinto a laser marker ML9110 (manufactured by Keyence Corp., CO₂ laser, power consumption: 450 VA) while adjusting the laser output and feed rate, and the liner was then cut into a square of 32 mm x 32 mm to produce Sample Nos. 1 to 7 having various groove forms shown in Table 1.

### (Example 2)

### Production of Patch preparation with Liner where Groove is Formed:

### 1. Preparation of Pressure-Sensitive Adhesive

In hexane, 30 Parts of low molecular-weight polyisobutylene (viscosity average molecular weight: 60,000, HIMOL 6H, produced by Nippon Petrochemicals Co., Ltd.) and 20 parts of high molecular-weight polyisobutylene (viscosity average molecular weight: 990,000, VISTANEX MML-80, produced by Exxon Chemical) were dissolved to prepare a polyisobutylene-based pressure-sensitive adhesive solution (solid content concentration: 30 wt%). To this solution, 6 parts of polybutene (viscosity average molecular weight: 1,260, HV-300F, produced by Nippon Petrochemicals Co., Ltd.) and 14 parts of alicyclic petroleum resin (softening point: 100°C, ARKON P-100, produced by Arakawa Chemical Industries, Ltd.) were added and dissolved with stirring. To the resulting solution, a hexane solution of tulobuterol (TBL) was added to have a TBL content of 10 wt% as a drug in the pressure-sensitive adhesive layer, and the mixture was thoroughly stirred to prepare a TBL pressure-sensitive adhesive solution (solid content concentration: 26 wt%).

### 2. Production of Coated Product

The TBL pressure-sensitive adhesive solution was coated on the release-treated surface of a PET-made liner having a thickness of 75 µm and a width of 560 mm as a liner (with one surface being release-treated, bending resistance: about 110 mm) by using a coating machine equipped with a comma coater and a three-zone drying tower to form a pressure-sensitive adhesive layer having a width of 530 mm and a dry thickness of 20 µm. The pressure-sensitive adhesive layer was laminated to the PET film side of a laminate film (produced by Kohjin Co., Ltd.), as the backing, of PET film (thickness: 6 µm) and PET non-woven fabric (20 g·m⁻²) to produce a coated product (laminated stock).

### 3. Aging and Scouring

The coated product was subjected to ripening (aging) at 20°C for 7 days to obtain a stock roll of a patch preparation. The stock roll was cut using a slitter to prepare a 38 mm-wide stock for test.

### 4. Treatment for Processing Groove

### (1) Laser Processing

In Sample Nos. 8, 9 and 11 to 14 of Table 2, a groove was formed by laser processing on the liner surface of the stock for test.

Grooves of various configurations shown in Table 2 were formed on the liner surface by using a laser cutting device (manufactured by Daisho Kagaku Kikai Kogyo K.K.) having incorporated thereinto a laser marker ML9110 (manufactured by Keyence Corp., CO₂ laser, power consumption: 450 VA) while adjusting the laser output and feed rate. The stock was cut into a square of 32 mm x 32 mm to produce a patch preparation.

### (2) Die Roll Processing

A die cutting device with a blade having a tip angle of about 30° was used. A die roll was pressed from the liner surface on the stock under conveyance to effect cutting to an extent of slightly intruding into the pressure-sensitive adhesive layer, and the stock was taken up. Thereafter, a patch preparation was produced in the same manner as in the laser processing.

### 5. Packaging of Patch preparation

The patch preparation was hermetically packaged in a packaging material having an outer surface formed of a 12 µm-thick PET film and an inner surface formed of a 30 µm-thick Hightoron resin.

### (Example 3)

### Storage Test:

The packaged patch preparation was stored at 40°C and a relative humidity of 75% for one, two, three or six months.

### (Test Example 1)

### Evaluation Method:

As for the cross-sectional shape of the groove, the width at the top of the groove (Tables 1 and 2) was determined by observation through a microscope (manufactured by Keyence Corp.). The thickness of the connected part (Tables 1 and 2) was calculated by determining the groove depth in the same manner as above and subtracting the obtained value from the film thickness. Incidentally, in Table 1, "-" indicates that the determination is unnecessary or impossible.

As for the effects (Tables 1 and 2), the PET film by itself (Table 1) and the patch preparation (Table 2) each was double-folded along the groove part and evaluated with an eye by rating A when complete liner division could be effected, B when complete liner division failed, and C when liner division could not be achieved.

The TBL content in the patch preparation after storage was measured by gas chromatography. The drug content after each storage time was expressed by a relative value to the TBL amount before storage, which was taken as 100 wt%.

### (Test Example 2)

### Results:

The evaluation results are shown in Tables 1 to 3 and Fig. 3.

In Table 1, the evaluation results of the liner by itself are shown. In all samples, the cross-sectional shape of the groove was a substantially V-shape. As apparent from Table 1, when the PET film by itself was folded along the groove formed in the film, the liner division could be effected (effect: A) in samples where the thickness Y of the connected part is less than 1/2 of the film thickness (that is, the groove depth is 1/2 or more of the film) and the bending resistance is 70 mm, 140 mm or 150 mm (Nos. 3, 5 and 7). However, the liner division could not be achieved in samples where the thickness Y of the connected part is 1/2 or more of the film thickness (Nos. 2, 4 and 6). Incidentally, groove formation was impossible in Sample No. 1. The results shown in Table 1 are for the evaluation of the PET film by itself, but the same results were expected also as a patch preparation.

**Table 1**

| No. | Bending Resistance (thickness) | Cross-sectional Shape of Groove | | Effect |
|---|---|---|---|---|
| | | Thickness Y of Connected Part (µm) | Width at Groove Top (µm) | |
| 1 | 40 mm (25 µm) | - | - | C |
| 2 | 70 mm (50 µm) | 27.0 | 150 | C |
| 3 | ditto | 21.0 | 170 | A |
| 4 | 140 mm (100 µm) | 56.5 | 180 | C |
| 5 | ditto | 32.0 | 190 | A |
| 6 | 150 mm or more (150 µm) | 75.5 | 200 | C |
| 7 | ditto | 60.0 | 210 | A |

In Table 2, the evaluation results of the patch preparation are shown. In all samples, the cross-sectional shape of the groove was a substantially V-shape. Also in Table 2 showing the evaluation results for the patch preparation, the same tendency was observed. When the patch preparation was folded along the groove formed in the liner having a liner bending resistance of 110 mm, the liner division could be effected (effect: A) in sample Nos. 8, 9, 10 and 12 where the groove depth is 1/2 or more of the liner thickness. In Sample No. 13 where the groove depth was slightly shallower than 1/2 of the liner thickness, the liner division was incomplete, and in Sample Nos. 11, 14 and 15, the liner division could not be achieved. In Sample Nos. 9 and 10 where the groove reached the pressure-sensitive adhesive layer, there was concern about the effect on the pressure-sensitive adhesive layer.

**Table 2**

| No. | Processing Method | Cross-sectional Shape of Groove | | Effect | Temporal Stability of Drug |
|---|---|---|---|---|---|
| | | Thickness Y of Connected Part (µm) | Width at Groove Top (µm) | | |
| 8 | laser | 25.0 | 170 | A | ⊚ |
| 9 | laser | 0.0 | 190 | A | △ |
| 10 | die cut | 0.0 | 100 | A | ○ |
| 11 | laser | 0.0 | 200 | C^{(*1)} | - |
| 12 | laser | 28.5 | 160 | A | - |
| 13 | laser | 37.0 | 140 | B | - |
| 14 | laser | 48.0 | 100 | C^{(*2)} | - |
| 15 | die cut | 42.0 | 80 | C^{(*2)} | - |

| | | | | | |
|---|---|---|---|---|---|
| (*1) The groove reached the backing. (*2) The liner division was impossible. | | | | | |

As seen in Table 3 and Fig. 3, when the patch preparation of Sample Nos. 8, 9 and 10 was stored, the drug content in the pressure-sensitive adhesive layer tended to decrease with time. Reviewing the data for after storage of six months, in Sample Nos. 8 and 9 where the thickness of the connected part of the groove is 0 µm, the drug content was greatly decreased. Even in Sample No. 8 where the groove did not reach the pressure-sensitive adhesive layer, the drug content was reduced due to volatilization of the drug from the edge part of the patch preparation, but the reduction rate was greatly suppressed. Also, there was a tendency that as the width at the top of the groove is smaller, the drug content less decreases.

**Table 3**

| No. | Change of Drug Content (%)with Time (month) | | | | |
|---|---|---|---|---|---|
| | 0 | 1 | 2 | 3 | 6 |
| 8 | 100.0 | 96.7 | 95.1 | 93.3 | 86.6 |
| 9 | 100.0 | 95.3 | 93.5 | 91.6 | 82.6 |
| 10 | 100.0 | 95.6 | 94.3 | 92.4 | 84.8 |

While the invention has been described in detail and with reference to specific embodiments thereof, it will be apparent to one skilled in the art that various changes and modifications can be made therein without departing from the spirit and scope thereof.

This application is based on Japanese Patent Application (Patent Application No. 2006-192346) filed on July 13, 2006, the entirety of which is incorporated herein by reference.

Also, all references cited herein are incorporated by reference herein in their entirety.

### INDUSTRIAL APPLICABILITY

According to the present invention, there is provided a patch and a patch preparation, where the pressure-sensitive adhesive layer has high temporal stability without causing contamination, mechanical damage or thermal damage in the pressure-sensitive adhesive layer until immediately before use and where the shape as a patch and a patch preparation can be easily maintained and the liner can be easily separated so as to ensure excellent handleability.

## Claims

1. A patch comprising a backing, a pressure-sensitive adhesive layer formed on one surface of the backing, and a liner having a thickness of T and laminated on the pressure-sensitive adhesive layer, wherein the liner has a groove formed from the surface opposite to the surface on which the pressure-sensitive adhesive layer is laminated and having a depth of from T/2 to less than T, the groove has a planar shape which enables the liner to be divided into two or more liner pieces by the groove, and the liner has a bending resistance before forming the groove of 50 mm or more.

2. The patch according to claim 1, wherein the groove has a width at the bottom of 200 µm or less.

3. The patch according to claim 2, wherein the groove has a cross-sectional shape of a substantially U-shape or a substantially V-shape.

4. A patch preparation comprising the patch according to any one of claims 1 to 3, wherein the pressure-sensitive adhesive layer contains a drug.
